# EUROPEAN PATENT SPECIFICATION

(11) **EP 2 054 116 B1**
(45) Date of publication and mention of the grant of the patent: **24.11.2010**
(21) Application number: 07841099.0
(22) Date of filing: 20.08.2007
(51) Int. Cl.: A61M 25/09

(54) **LOOP TIP WIRE GUIDE**
SCHLEIFENSPITZENDRAHTFÜHRUNG
FIL DE GUIDAGE AVEC EMBOUT À BOUCLE

(30) Priority: 25.08.2006 US 840214 P
(43) Date of publication of application: 06.05.2009
(73) Proprietor: Wilson-Cook Medical Inc., Winston-Salem, NC 27105-4191 (US); Cook Incorporated, Bloomington IN 47404 (US)
(72) Inventor: CARTER, Matthew, P., Dobson, NC 27017 (US); HARDIN, David, M., Winston-salem, NC 27106 (US); MELSHEIMER, Jeffry, S., Springville, IN 47462 (US); SURTI, Vihar, C., Winston-salem, NC 27106 (US)
(74) Representative: Garratt, Peter Douglas
(86) International application number: PCT/US2007/076305
(87) International publication number: WO 2008/024707

(56) References cited:
- WO-A-01/03764
- FR-A- 2 511 600
- US-A1- 2006 100 545

## Description

This application claims the benefit of U.S. Provisional Application No. 60/840,214 filed August 25, 2006.

### BACKGROUND

1. Technical Field

This invention relates to wire guides used in the placement of medical devices, and more particularly to wire guides having a loop tip.

2. Background Information

Wire guides are elongate flexible members used to provide a path along which another medical device can be moved. The path provided by the wire guide can be used to navigate another medical device, such as a catheter through a body vessel. The use of wire guides to define such a path is known in the art. Briefly, a wire guide is navigated through a body lumen toward a point of treatment. Once positioned within the lumen, a therapeutic or diagnostic device, (i.e., a catheter) may be advanced over the wire guide to the target site and the desired therapeutic or diagnostic steps may be performed. The wire guide provides an established path for placing other devices and eliminates the need for performing delicate navigation procedures for each device passed into the body lumen, for example when additional procedures are performed.

During placement of the wire guide, an operator must navigate the wire guide through a tortuous pathway in the body lumen due to the presence of natural bends and/or curves, or unnatural impediments, such as tumors, build-ups, and /or strictures. The presence of a tortuous path may make navigation of a wire guide through the path difficult, for example, the tip of the wire guide may get bent away from the desired path or caught in a stricture, or in some cases even perforate the wall of the lumen, etc. making further navigation into the lumen difficult or impossible.

The prior art contains many examples of wire guides having straight, flexible tips intended to aid in navigation of tortuous body lumens. The presence of a straight tip, however, may make navigation more difficult. For example, upon encountering an impediment, the straight tip may bend (reflex) into the lumen wall and become caught. Contact of the straight tip with the lumen wall may prevent the wire guide from advancing further into the lumen as well as possibly damaging the lumen wall.

Reference is directed to WO 01/03764 in which there is provided a catheter comprising an elongated body having an elongated body having a proximal end and a distal end, wherein at the distal end of the catheter, a cap is provided, attached to the distal end, the cap is provided with a passage or a loop adapted to receive a guide-wire therethrough. In another preferred embodiment, a catheter is provided with external coating at its distal end, wherein the coating is provided with longitudinal cleavage, and a perforation substantially opposite the cleavage so as to allow guide-wire to be passed through the cleavage and be threaded through the perforation.

What is needed is an improved wire guide tip for navigating a tortuous body lumen where the improved wire guide includes a loop tip configured for facilitating navigation and reducing trauma to the lumen wall during advancement of the wire guide.

### BRIEF SUMMARY

The scope of the present invention is set forth in the appended claims. The various preferred embodiments provide significant improvements and advantages over conventional straight wire guide tips.

An elongate medical device configured for navigation through a bodily lumen is described. The device as further disclosed in claim 1 includes an elongate shaft having a proximal portion and a distal portion. The distal portion includes a first interlocking connector. The device further includes a loop portion operably connected to the distal portion. The loop portion indudes a second interlocking connector configured for connecting to the first interlocking connector.

An elongate medical system is described. The system includes a catheter and a wire guide extending from a distal portion of the catheter. The wire guide includes an elongate shaft having a proximal portion and a distal portion. The distal portion of the shaft includes a first interlocking connector. The wire guide further includes a loop portion having a second interlocking connector configured for connecting to the first interlocking connector on the shaft.

A method of manufacturing a wire guide having a loop tip is described and is not part of the invention. The method includes forming an elongate shaft having a proximal portion and a distal portion. The distal portion has a first interlocking connector. The method further includes forming a loop structure having a second interlocking connector. The first interlocking connector of the shaft is connected to the second interlocking connector of the loop.

The foregoing paragraphs have been provided by way of general introduction, and are not intended to limit the scope of the following claims. The presently preferred embodiments, together with further advantages will be best understood by reference to the following detailed description taken in conjunction with the accompanying drawings.

### BRIEF DESCRIPTION OF THE DRAWINGS

Embodiments of the present invention will now be described by way of example with reference to the accompanying drawings, in which:

FIG. 1 is a partial side view of a wire guide having a loop tip according to the present invention;

FIG. 2A is a partial side view of an embodiment of a loop portion having a receiving structure at a loop interlocking connector;

FIGS. 2B-2E are partial side views of alternative connections at a shaft interlocking connector;

FIG. 3A is a partial cross-sectional side view of the wire guide shown in FIG. 1 showing the interlocking connectors prior to connection;

FIG. 3B is a partial side view of the an alternative embodiment of the interlocking connectors shown in FIG. 3A;

FIG. 3C is a partial side view of an alternative embodiment of the interlocking connectors shown in FIG. 3A;

FIG. 4A is a partial side view of an alternative embodiment of the wire guide shown in FIG. 1;

FIG. 4B is a partial perspective view of a section of the loop tip of the wire guide shown in FIG. 4A;

FIG. 4C is a partial side view of an alternative connection for the wire guide shown in FIG. 4A;

FIG. 5A is a partial side view of an alternative embodiment of the wire guide shown in FIG. 1;

FIG. 5B is a partial perspective view of a section of the loop tip of the wire guide shown in FIG. 5A;

FIG. 6A is a partial side view of an alternative embodiment of the loop tip wire guide;

FIG. 6B is a partial side view of an alternative embodiment of the loop tip wire guide;

FIG. 7A is a partial side view of an alternative embodiment of the wire guide shown in FIG. 1;

FIG. 7B is a partial side view of m alternative embodiment of the wire guide shown in FIG. 7A;

FIG. 8 is a partial side view of the wire guide illustrating an alternative method of formation of the loop tip;

FIG. 9A is a perspective view of tubing used to form a loop;

FIG. 9B is a perspective view of a loop formed from the tubing shown in FIG. 9A;

FIG. 10A is a side view of a catheter comprising a handle with a proximal portion of a wire guide extending from an exit port;

FIG. 10B is a side view of a proximal portion of the catheter shown in FIG. 10A with a wire guide disposed through a lumen thereof;

FIG. 10C is a side view of a proximal portion of an alternative embodiment of the proximal portion shown in FIG. 10B;

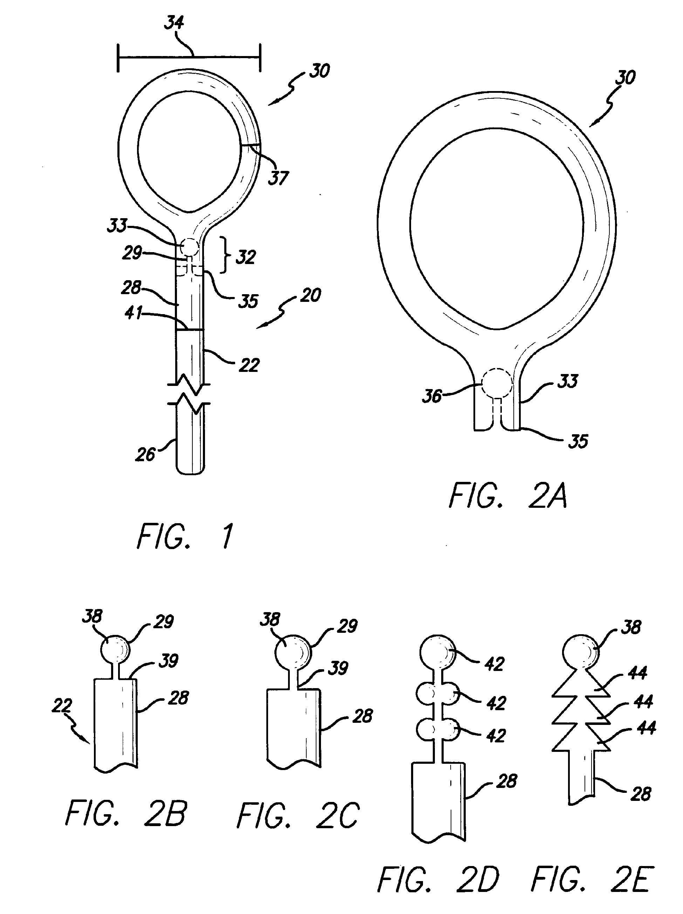

FIG. 1 IA illustrates the wire guide of the present invention entering the common bile duct; and

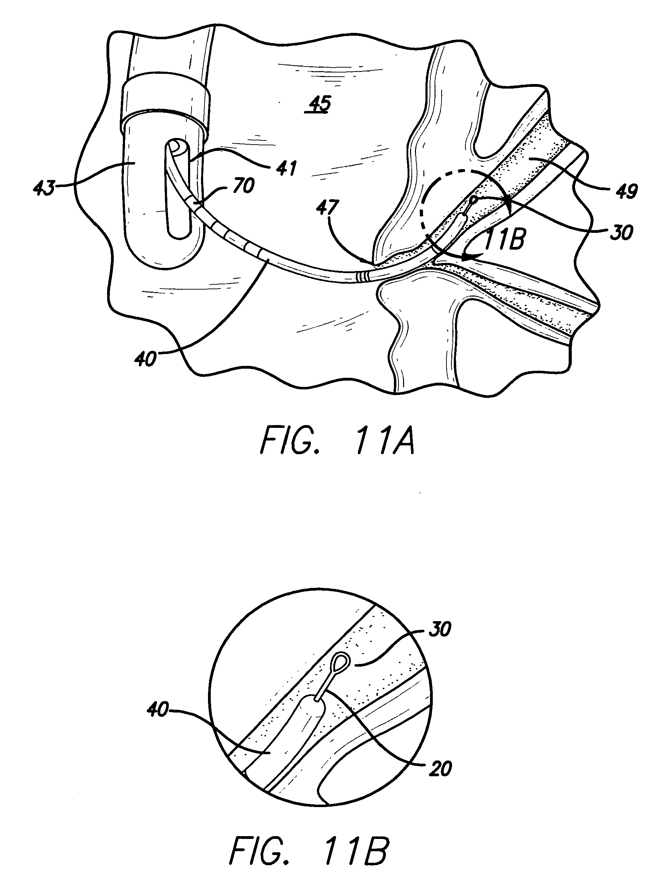

FIG 11B is an enlarged view of the loop tip shown in FIG. 11A.

### DETAILED DESCRIPTION OF THE INVENTION

The invention is described with reference to the drawings in which like elements are referred to by like numerals. The relationship and functioning of the various elements of this invention are better understood by the EolIowing detailed description. However, the embodiments of this invention as described below are by way of example only, and the invention is not limited to the embodiments illustrated in the drawings. It should also be understood that the drawings are not to scale and in certain instances details have been omitted, which are not necessary for an understanding of the present invention, such as conventional details of fabrication and assembly.

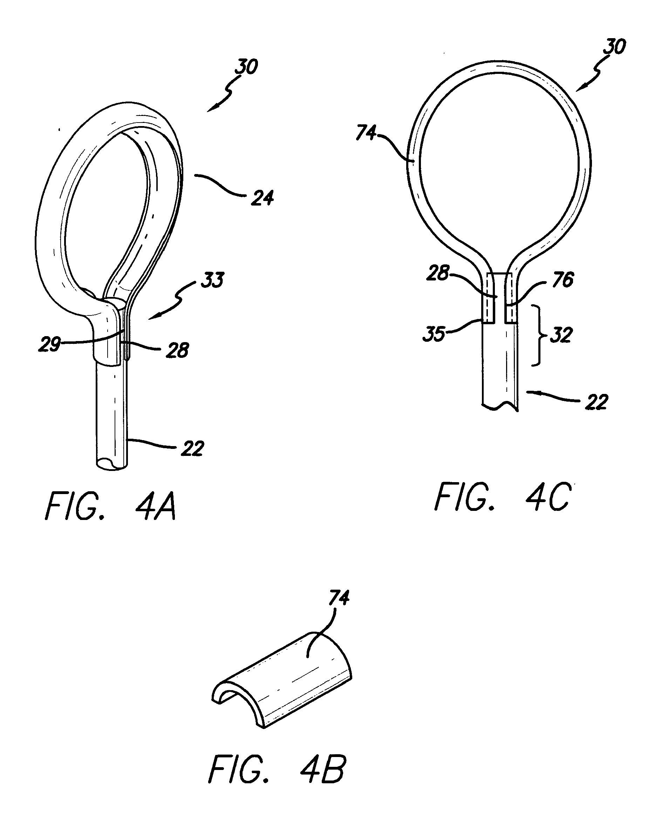

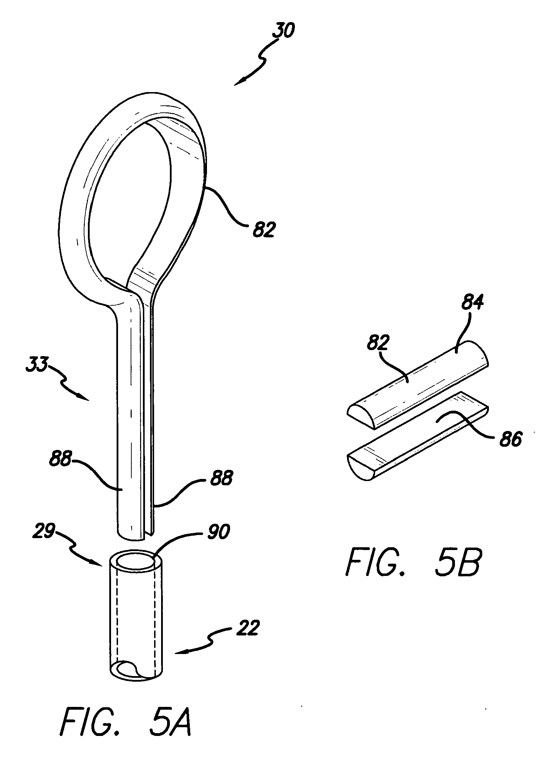

FIG. 1 illustrates a wire guide 20 according to the present invention. The wire guide 20 includes an elongate shaft 22 having a proximal portion 26, a distal portion 28 and a loop 30. The distal portion 28 includes a first connector 29 that is connectable to the loop 30 of the wire guide 20. The loop 30 may include a loop connector 33 that is sized and shaped to fit together with the shaft connector 29. The connectors 29 and 33 may have any size and shape that can be engaged together to join the shaft 22 and the loop 30 at a connecting structure 32 of the wire guide 20 as will be discussed in greater detail below. The first connector 29 is releasably interlocking with the loop connector 33. As shown, the loop 30 is generally curvilinear in shape, although other shapes are possible. In some embodiments, the loop is generally oval in shape. Additional shapes include, but are not limited to round, arched, and parabolic. The cross-sectional shape of the loop 30 or portions thereof, may be any shape. For example, the cross-sectional shape of the loop 30 may be rectangular, arched rectangular, circular, teardrop, oval and the like. See for example FIGS. 4B and 5B. The thickness of the material and the type of material used to form the loop 30 may be varied to provide flexibility for the loop 30 as will be understood by one skilled in the art. The loop 30 may be deformable as the loop 30 moves through a tortuous body lumen, including past any impediments that may be in the lumen such as a tumor or a sphincter. The shape of the loop 30 refers to the general shape in a pre-advancement state. The loop 30 has a diameter 34 measured at the widest portion of the loop 30. The loop 30 may also be adapted for application to any elongate medical device, such as a catheter. In some embodiments, the proximal portion 26 of the wire guide 20 has a diameter 41 that is smaller than the diameter 34 of the loop 30.

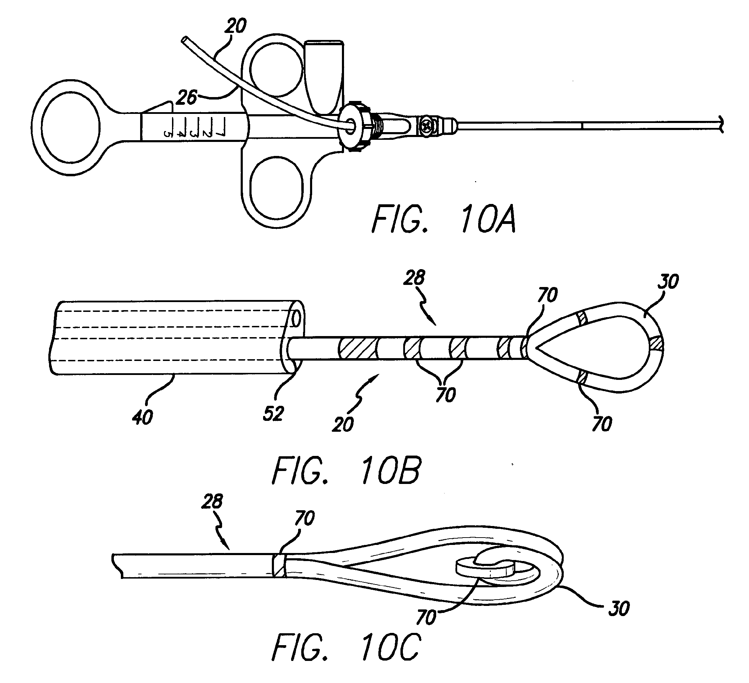

As shown in FIG. 1, the connecting structure 32 is formed where the connector 29 of the shaft 22 joins the connector 33 of the loop 30. The connecting structure 32 may have a smooth outer surface 35 for facilitating passage through the body lumen. Exemplary connecting configurations are discussed below, however any configuration for connecting the loop 30 and the shaft 22 may be used. The connecting configurations where the first connector 29 interlocks with the second connector 33 is snap-fit. In some embodiments, the loop 30 may be completely removed from the shaft 22 after the loop 30 has traveled to the desired position in the bodily lumen. For example, the loop 30 may be removed by retracting the loop 30 back toward an end 53 of a catheter 40 so that contact of the loop 30 against the end 53 detaches the loop 30 from the shaft 22 (catheter shown in FIG 10B). The loop 30 configuration may be designed so that the loop is removable by pulling the loop 30 back against the catheter, for example, using a ball and socket configuration. The loop 30 may also include a coating (discussed below) over the connection between the loop 30 and the shaft 22 that is absorbable and is made to degrade after a desired amount of time after exposure to bodily fluids. The loop 30 may be bioabsorbable and/or may be removed in the gastrointestinal tract where the loop 30 may pass through and be eliminated.

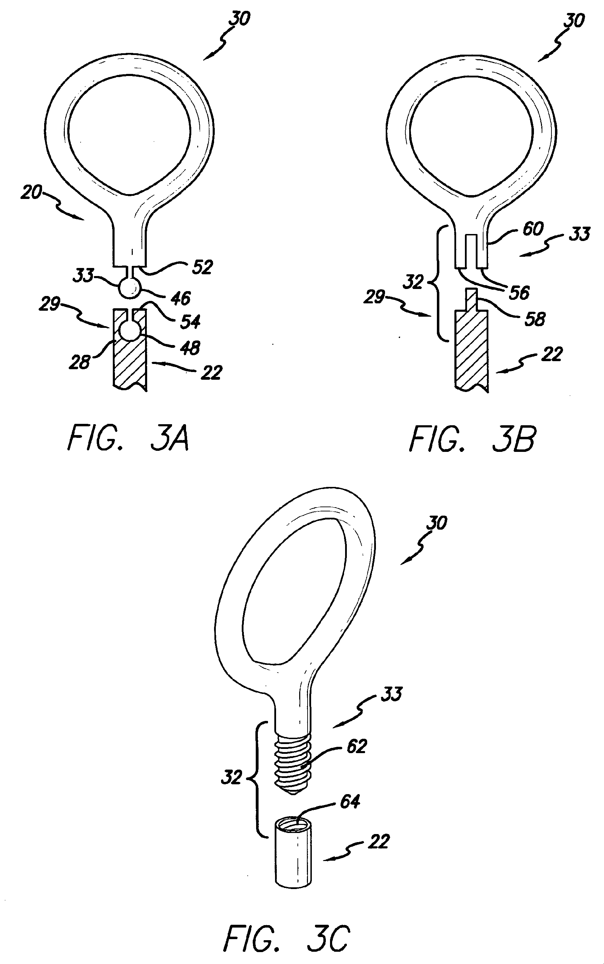

FIGS. 2A-2E and 3A-3C illustrate embodiments showing different sizes, shapes and forms for providing the connector 29 on the shaft 22 and the connector 33 on the loop 30. For example, FIG. 2A illustrates the loop 30 having the connector 33 formed from a spherically-shaped receiving structure 36 that is sized and shaped to engage with a connector 29 that is formed from a spherically-shaped protruding tip 38 extending on a stem 39 (FIG. 2B), e.g. a ball and socket connection. This configuration is designed for snap-fit connection with the receiving structure 36 being sufficiently flexible to open to receive the protruding tip 38 and to close when the tip 38 is in the receptacle 36.

Alternative configurations for the connector 29 are shown in FIG. 2C-2E that will join together with a correspondingly shaped connector 33 on the loop 30 (not shown). For example, as shown in FIG. 2C, the stem 39 may be extended further distally as compared to the stem 39 shown in FIG. 2A and may also include the spherically-shaped protruding rip 38. The connector 33 of the loop 30 will include a correspondingly shaped receptacle. As shown in FIG. 2D, the connector 29 of the shaft 22 may also be formed from a plurality of spherically-shaped protrusions 42 and have a corresponding number of spherically-shaped receiving structures formed in the connector 33 of the loop 30. In another configuration, shown in FIG. 2E, the connector 29 may be formed from a plurality of conically shaped protrusions 44 and may further include the spherically-shaped protruding tip 38. As one skilled in the art will understand, any shape and number of protrusions may be formed on the connector 29 to permit the connector 29 to be joined with a correspondingly shaped receptacle formed on in the connector 33 of the loop 30.

FIGS. 3A-3C illustrate alternative configurations for the connector 29 of the shaft 22 and the connector 33 of the loop 30. For example, as shown in FIG. 3A, the loop 30 includes a connector 33 having a spherically-shaped protrusion 46 and the shaft 22 includes a connector 29 having a spherically-shaped receptacle 48. The protrusion 47 and the receptacle 48 fit together so that a proximal portion 52 of the loop 30 abuts a distal portion 54 of the shaft 22 forms the outer surface 35 of the wire guide 20. Similarly, the loop 30 and the shaft 22 may be connected together by protrusions 56 extending from the loop 30 that interlocks with one or more protrusions 58 extending from the shaft 22 as shown in FIG. 3B. The protrusions 56 and 58 are sized and shaped to fit together at the connecting structure 32. The protrusions 56 and 58 may be any shape and may include a curved outer surface 60 to form a smooth connecting structure 32. FIG. 3C illustrates another alternative configuration for connecting the loop 30 to the shaft 22. The loop 30 includes a connector 33 having an externally threaded proximal portion 62. The shaft 22 includes an interlocking connector 29 having an internally threaded portion 64 for joining with the proximal portion 62 of the loop at the connecting structure 32. Alternative embodiments may include an externally threaded connector 29 on the shaft 22 and an internally threaded connector 33 on the loop 30. In some embodiments, the connector 29 of the shaft 22 and the connector 33 of the loop 30 will join together and form the smooth outer surface 35 (as shown in FIG. 1) to facilitate passage of the wire guide 20 through the body lumen.

In some embodiments, the loop 30 may be externally connected to the distal portion 28 of the shaft 22. As shown in FIGS. 4A-4C, the loop 30 may be formed from a curved, rectangular ribbon member 74 that is shaped to curve around the connector 29 on the shaft 22 of the wire guide 20 and form a connector 33. A portion of the ribbon member 74 is shown in FIG. 4B illustrating the curve of the ribbon member itself. The curved shape of the ribbon member 74 forming the loop 30 may help navigating the tortuous lumen and provide flexibility as well as enough rigidity to resist buckling while the wire guide 20 moves forward. The loop 30 shown in FIG. 4C may be configured to curve around the distal portion 28 of the shaft 22 where a portion 76 of the distal portion 28 is recessed with respect to the outer surface 35 at the connector 29. In the embodiment shown in FIG. 4C, the outer surface 35 of the wire guide 20 is configured to provide a smooth surface.

In some embodiments, the loop 30 may be formed from a semi-circularly shaped member 82 where the curved portion 84 of the semicircle extends outward and the flattened portion 86 faces inward. The loop 30 may be formed by positioning end regions 88 of the semicircle member 82 with the flattened portions 86 against each other wherein the loop 30 extends between the end regions. As shown in FIG. 5A, the flattened portion 86 of the end regions 88 may be abutted against each other to form the connector 33 and inserted into an opening 90 at the connector 29 in the shaft 22. FIG. 5B illustrates an exploded view of adjacent flattened portions 86 that may be positioned against each other to form the loop 30 shown in FIG. 5A. The opening 90 may be sized and shaped to receive the end regions 88 of the loop 30 to form the wire guide 20. Alternatively, the end regions 88 may be connected to the shaft 22 using any configuration.

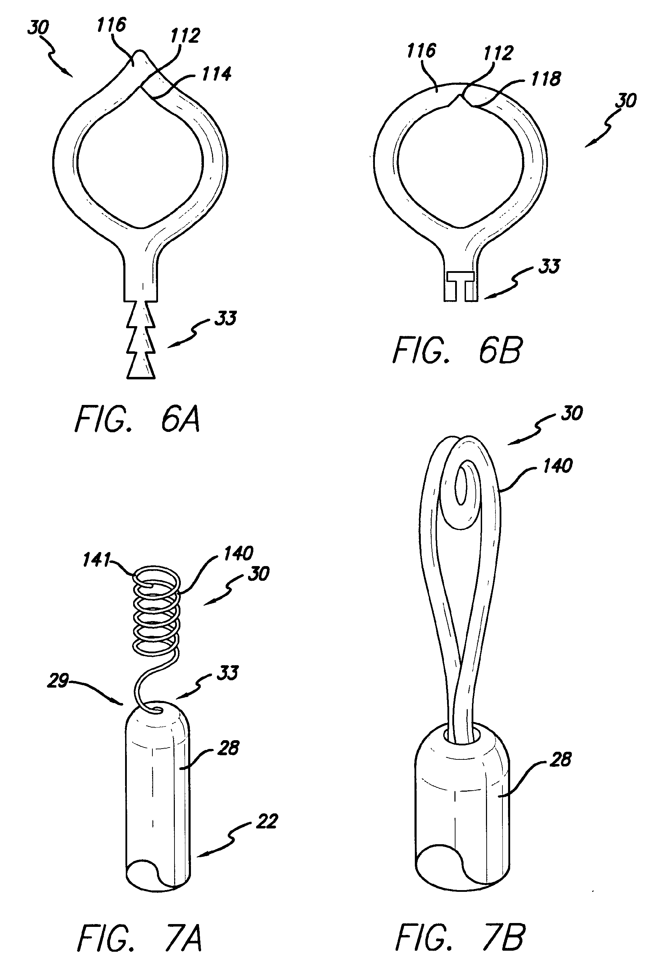

In some embodiments, the loop 30 may include a folding region 112 to facilitate folding of the loop 30, for example, for withdrawing the loop 30 into a catheter. As shown in FIGS. 6A, the folding region 112 may be provided by forming an arch 114 at a leading edge 116 of the loop 30 in the direction of navigation. The folding region 112 may also be a notch 118 cut into the leading edge 116 of the loop 30 as shown in FIG. 6B. In these embodiments having the folding region 112, the arch 114 or notch 118 may be positioned so that the loop 30 moves forward through the body lumen by flexing with the contours of the lumen and maintaining the loop configuration without folding. When the loop 30 is withdrawn from the body lumen and into a catheter (see FIGS. 10A and 10B), the loop 30 may fold into a narrower configuration by bending inwardly at the arch 114 or the notch 118 for facilitating withdrawal of the wire guide 20. The folding region 112 may be provided with any of the loop configurations described herein. The folding region 112 may be provided by including a region in the loop 30 that is more flexible at the folding region 112 in comparison to the surrounding loop portions. For example, the material forming the folding region 112 may be more flexible, have a reduced thickness, or serrations or cut away portions on the inner surface of the loop 30.

In some embodiments, the loop 30 may be provided as a helical coil configuration 140 as shown in FIG. 7. The coil 140 may be provided as an extension from an inner coil of the wire guide 20 (not shown) or the coil 140 may be provided separately and connected to the wire guide 20 using any method known to one skilled in the art. The coil 140 may be resiliently compressible for navigating though the body lumen. An end 141 of the coil 140 may be bent inwardly towards the center of the coil 140 so that the end 141 does not get caught in the lumen as the wire guide is being extended or retracted as shown in FIG. 7A. In some embodiments, both ends 141 of the coil 140 may be connected to the distal portion 28 of the shaft 22 as shown in FIG. 7B.

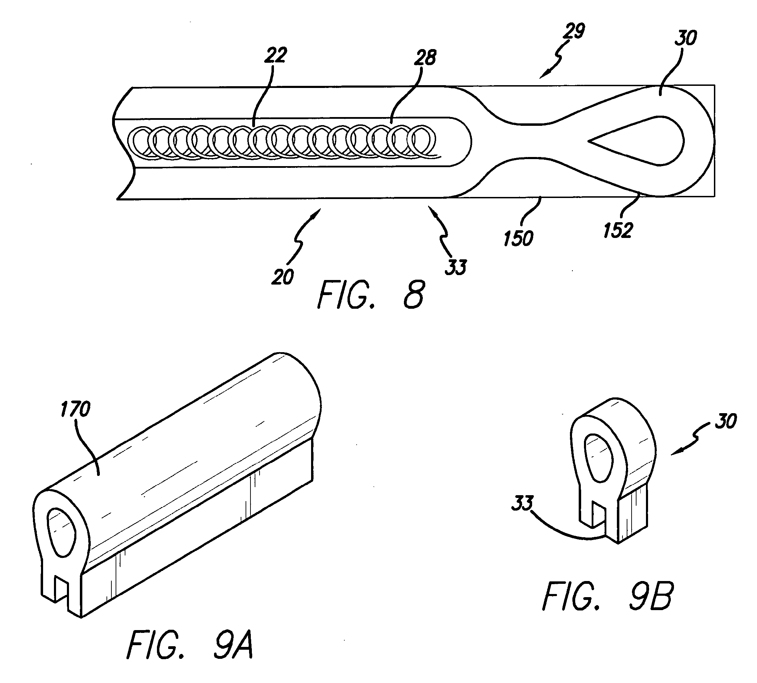

The loop 30 may also be formed as a die cut structure as shown in FIG. 8. The loop 30 having the connector 33 may be insert-molded, for example, from a polymer that has been molded onto the connector 29 at the distal portion 28 of the shaft 22 of the wire guide 20 connecting the loop 30 to the shaft 22. The polymer may form the connector 33 by being molded onto the connector 29 of the wire guide 20 within a mold 150. The mold 150 may include a form 152 to shape the polymer into the loop configuration. Alternatively, the polymer may be formed and solidified in the mold 150 and then the loop 30, including the connector 33, may be cut from the polymer using techniques known to one skilled in the art.

In some embodiments, the loop 30 may be formed from tubing 170 that may be molded or extruded in the desired shape for the loop 30. For example, as shown in FIG. 9A, the profile of the loop 30 includes the connector 33 shaped to fit with the connector 29 of shaft 22. The loop 30 may be formed in any shape with any shape connector 33. The tubing used to form the loop 30 may be injection molded, dipped, rotational molded, slush molded, fiber spun, blow molded, extruded or over molded to the desired form. The length of tubing 170 may be sectioned into individual loops 30 shown in FIG. 9B by cutting the tubing at the desired thickness. The loop 30 may be connected to the connector 29 of the shaft 22 by any method known to one skilled in the art. The tubing may be formed from any material, for example, PTFE. A coating layer may be included over the loop 30 at the connecting structure 32 to form the smooth outer surface 35.

Any method may be used to connect the connector 29 of the shaft 22 to the connector 33 of the loop 30. As described above, the connectors 29 and 33 may be sized and shaped to releasaby interlock together the connectors 29 and 33 for example, by forming a snap-fit connection between the portions or by threading together the respective threads. In addition or as an alternative, the connectors 29 and 33 may be connected by bonding, including, but not limited to adhesive bonds and solder bonds, welding and molding. Combinations of these methods may also be used.

Any suitable material can be used for the wire guide 20 and portions thereof. The material chosen need only be biocompatible, or made biocompatible, and able to be formed into the structures described herein. Exemplary materials will also be compliant, elastic, resilient and have shape memory. Portions of the wire guide 20, such as the loop 30 and the shaft 22 may be made from different materials or the same materials. Examples of suitable materials include, but are not limited to stainless steel, tantalum, nitinol; gold, silver, tungsten, platinum, inconel, cobalt-chromium alloys and iridium, all of which are commercially available metals or alloys used in the fabrication of medical devices. Portions of the wire guide may be formed from a medically-acceptable polymer. For example, exemplary polymers include, but are not limited to, cellulose acetate, cellulose nitrate, silicone, polyethylene, high density polyethylene, polyethylene teraphthalate, polyurethane, polytetrafluoroethylene (PTFE), polyamide, polyester, polyorthoester, polyvinyl chloride (PVC), polypropylene, acrylonitrile-butadienestyrene (ABS), polycarbonate, polyurethane, nylon silicone, and polyanhydride.

Portions of the wire guide 20 may also be made from a bioabsorbable material. A number of bioabsorbable homopolymers, copolymers, or blends of bioabsorbable polymers are known in the medical arts. These include, but are not necessarily limited to, polyesters including poly-alpha hydroxy and poly-beta hydroxy polyesters, polycaprolactone, polyglycolic acid, polyether-esters, poly(p-dioxanone), polyoxaesters; polyphosphazenes; polyanhydrides; polycarbonates including polytrimethylene carbonate and poly(iminocarbonate); polyesteramides; polyurethanes; polyisocyantes; polyphosphazines; polyethers including polyglycols polyorthoesters; expoxy polymers including polyethylene oxide; polysaccharides including cellulose, chitin, dextran, starch, hydroxyethyl starch, polygluconate, hyaluronic acid; polyamides including polyamino acids, polyesteramides, polyglutamic acid, poly-lysine, gelatin, fibrin, fibrinogen, casein, and collagen. For example, when the loop 30 is die-cut as described above, the loop 30 may be made from PTFE.

The wire guide 20, or portions thereof, may comprise a wire, a tubular member or a sheet of material. Further, the wire guide 20 or portions thereof may be formed from a series of layers, or as a coated core structure. For example, in one embodiment, the shaft 22 may comprise a nitinol core with a PTFE covering. The loop 30 may also be formed of nitinol and may include a PTFE covering. Portions of the loop 30 may also be reinforced for example, by providing additional layers of materials or stronger material from the list above, in or to prevent kinking. Any reinforcing materials will still allow the loop 30 to flex as it navigates through the lumen to the treatment site.

A variety of shapes and sizes of the shaft 22 and the loop 30 may be used, and these can both be optimized based on particular applications. The dimensions of the shaft 22 and the loop 30 will depend upon various factors, including the intended use of the wire guide 20 and the body lumens into which the wire guide 20 will be positioned. For a wire guide 20 intended to cannulate the common bile duct, suitable dimensions include a shaft diameter 39 of between approximately 0,4064 mm (0.016) inches and approximately 0,9652 mm (0.038) inches, and preferably comprises a diameter 39 of approximately 0,889 mm (0.035) inches. The distal portion diameter 37 forming the loop 30 of the wire guide 20 preferably has a diameter of between approximately 0.003 inches and approximately 0.010 inches, and preferably comprises a diameter of approximately 0.006 inches. When the loop 30 is ovoid in shape and delivered to the bile duct, the length of the loop 30 may be between approximately 4 and approximately 5 millimeters, and the width 34 at the widest portion of the loop 30 may be between approximately 2 and approximately 3 millimeters. One skilled in the art will recognize that other sizes and shapes are possible depending on the bodily location the wire guide 20 is configured to enter. For example, the loop 30 may also be configured to enter the colon, pancreas and esophagus that may require different sizes than described above. Any size and shape loop 30 may be used with the present invention.

Coatings may also be applied to at least a portion of the wire guide 20. The coating(s) may be applied by dipping, molding, spraying, heat shrinking or extrusion of a suitable coating material, such as PTFE, polyolefin, polyvinyl chloride (PVC), polyester (PET) and fluorinated ethylene propylene (FEP) and/or other polymeric coatings, directly to the wire guide 20 or portions thereof. Bioabsorbable coatings may also be used.

In some embodiments, a thin heat shrinkable material may be used for the coating, such as PTFE. The heat shrinkable materials facilitate manufacturing while providing a lubricious coating, which facilitates navigation. In preferred embodiments, the thickness of the coating is between approximately 0.001 and 0.010 inches. In particularly preferred embodiments, the thickness of the coating is between approximately 0,0254 mm (0.001) and 0,127 mm (0.005) inches. In still more preferred embodiments, the thickness of the coating is between approximately 0.001 and 0.002 inches. These preferred thicknesses provide suitable coatings while not adding significantly to the overall thickness of the device.

Also, the wire guide 20 or portions thereof, with or without the coating described above, may be treated with a hydrophilic coating or hybrid polymer mixture, such as those based on polyvinyl puroladine and cellulose esters in organic solvent solutions. These solutions make the wire guide particularly lubricious when in contact with body fluids, which aids in navigation.

Radiopaque materials may be added in the coating. Also, radiopaque materials known in the art may be placed on the shaft 22 and the loop 30 and other portions of the wire guide 20. Several examples of suitable radiopaque materials and markers are known in the art, and any suitable material and/or marker can be utilized in the present invention. Common radiopaque materials include barium sulfate, bismuth subcarbonate, and zirconium dioxide. Other radiopaque elements include: cadmium, tungsten, gold, tantalum, bismuth, platinum, iridium, and rhodium. In one embodiment, iodine may be employed for its radiopacity and antimicrobial properties. Radiopacity is typically determined by fluoroscope or x-ray film. Radiopaque, physiologically compatible materials include metals and alloys selected from the Platinum Group metals, especially platinum, rhodium, palladium, rhenium, as well as tungsten, gold, silver, tantalum, and alloys of these metals. These metals have significant radiopacity and in their alloys may be tailored to accomplish an appropriate blend of flexibility and stiffness. They are also largely biocompatible. For example, a platinum/tungsten alloy, e.g., 8% tungsten and the remainder platinum may be used.

Operation of the wire guide 20 of the present invention is similar to conventional wire guides known in the art. The wire guide 20 may be provided to the operator preassembled with the wire guide 20 loaded into a catheter, such as the catheter 40 shown in FIGS. 10A and 10B-C. The catheter may be any catheter known to one skilled in the art, including, but not limited to, dual lumen, triple lumen catheters, balloon catheters, stent delivery catheters, cannulae, papillotomes and sphincterotomes, and the like. In some embodiments, the wire guide 20 may be back loaded into the lumen 52 so that the distal portion 28 including the loop 30 of the wire guide 20 extends distally from the catheter 40. Back loading refers to introduction of the proximal portion 26 of the wire guide 20 into the catheter 40 until the distal portion 26 extends out of a proximal guide wire exit (FIG. 10A). The wire guide 20 may be oriented in any direction when assembled into the catheter 40. For example, when the wire guide 20 is back loaded into a catheter 40 having an offset lumen, the wire guide 20 may be oriented so that the loop 30 is generally centered with respect to the catheter 40. As shown in FIGS. 10B and 10C, the wire guide 20 may include radiopaque portions 70 for orienting the wire guide 20 as the wire guide 20 is navigated through the passageways. The radiopaque portions 70 may be any type of radiopaque marker known to one skilled in the art.

The wire guide 20 may be advanced through the tortuous body lumen to the desired location and the catheter 40 may be advanced over the wire guide 20 following standard procedures known to one skilled in the art. FIG. 11A illustrates the wire guide 20 extending out an opening 41 of an endoscope 43. The endoscope 43 is positioned in the duodenum 45 and the wire guide 20 is shown entering the biliary tree 49 through the ampullary orifice (Papilla of Vater) 47. FIG. 11B is an enlargement of the wire guide 20 moving through a torturous path 44 within a body vessel 60. The loop 30 deforms slightly in response to the torturous path 44. This allows the wire guide 20 to continue navigating along the interior of the body vessel 60. The catheter 40 may be introduced over the wire guide 20 to the treatment location.

Any other undisclosed or incidental details of the construction or composition of the various elements of the disclosed embodiment of the present invention are not believed to be critical to the achievement of the advantages of the present invention, so long as the elements possess the attributes needed for them to perform as disclosed. The selection of these and other details of construction are believed to be well within the ability of one of even rudimentary skills in this area, in view of the present disclosure. Illustrative embodiments of the present invention have been described in considerable detail for the purpose of disclosing a practical, operative structure whereby the invention may be practiced advantageously. The designs described herein are intended to be exemplary only. The novel characteristics of the invention may be incorporated in other structural forms without departing from the scope of the invention. Unless otherwise indicated, all ordinary words and terms used herein shall take their customary meaning as defined in *The New Shorter Oxford English Dictionary, 1993 edition.* All technical terms shall take on their customary meaning as established by the appropriate technical discipline utilized by those normally skilled in that particular art area. All medical terms shall take their meaning as defined by *Stedman's Medical Dictionary, 27th edition.*

It is therefore intended that the foregoing detailed description be regarded as illustrative rather than limiting, and that it be understood that it is the following claims, including all equivalents, that are intended to define the scope of this invention.

## Claims

1. An elongate medical device (20) configured for navigation through a lumen; the device comprising:
an elongate shaft (22) comprising a proximal portion (26) and a distal portion (28), the distal portion comprising a first interlocking connector (29); and
a loop portion (30) comprising a second interlocking connector (33) configured for connecting with the first interlocking connector of the shaft;
wherein the second interlocking connector is configured for removably connecting with the first interlocking connector in a snap-fit connecting configuration.

2. The device according to claim 1, wherein one of the first interlocking connector and the second interlocking connector comprises a protrusion and the other of the first interlocking connector and the second interlocking connector comprises a receptacle.

3. The device according to claim 2, wherein the protrusion and the receptacle are spherically-shaped.

4. The device according to any of the preceding claims, wherein the loop portion comprises a folding region

5. The device according to any of the preceding claims, wherein at least a portion of the device comprises a bioabsorbable material.

6. The device according to any of the preceding claims, wherein at least a portion of the device comprises a polymer.

7. The device according to any of the preceding claims, wherein at least a portion of the device comprises nitinol.

8. The device according to any of the preceding claims, wherein at least a portion of the device further comprises a coating.

9. The device according to any of the preceding claims, wherein at least a portion of the device further comprises a radiopaque material.

10. The device according to any of the preceding claims, wherein the device comprises a coating covering at least the first interlocking connector and the second interlocking connector for forming a smooth outer surface.

11. The device any of the preceding claims, wherein the device comprises a wire guide.

12. The device any of the preceding claims, wherein the device is configured to be disposed in the gastrointestinal tract of a patient.

13. An elongate medical system comprising:
a catheter comprising a proximal portion and a distal portion; and
a wire guide according to claim 11 extending from the distal portion of the catheter.

## Patentansprüche

1. Längliche medizinische Vorrichtung (20), die zur Navigation durch ein Lumen konfiguriert ist; wobei die Vorrichtung Folgendes umfasst: einen länglichen Schaft (22), der einen proximalen Abschnitt (26) und einen distalen Abschnitt (28) umfasst, wobei der distale Abschnitt ein erstes verriegelndes Verbindungsglied (29) umfasst, und einen Schlaufenabschnitt (30) mit einem zweiten verriegelnden Verbindungsglied (33), das mit dem ersten verriegelnden Verbindungsglied des Schafts verbunden werden kann, worin das zweite verriegelnde Verbindungsglied über eine Schnappverbindung lösbar mit dem ersten verriegelnden Verbindungsglied verbunden werden kann.

2. Vorrichtung nach Anspruch 1, worin das erste verriegelnde Verbindungsglied oder das zweite verriegelnde Verbindungsglied einen Vorsprung umfasst, während das jeweils andere erste oder zweite verriegelnde Verbindungsglied eine Aufnahme umfasst.

3. Vorrichtung nach Anspruch 2, worin der Vorsprung und die Aufnahme kugelförmig sind.

4. Vorrichtung nach einem der vorhergehenden Ansprüche, worin der Schlaufenabschnitt einen Faltabschnitt umfasst.

5. Vorrichtung nach einem der vorhergehenden Ansprüche, worin zumindest ein Teil der Vorrichtung ein bioresorbierbares Material umfasst.

6. Vorrichtung nach einem der vorhergehenden Ansprüche, worin zumindest ein Teil der Vorrichtung ein Polymer umfasst.

7. Vorrichtung nach einem der vorhergehenden Ansprüche, worin zumindest ein Teil der Vorrichtung Nitinol umfasst.

8. Vorrichtung nach einem der vorhergehenden Ansprüche, worin zumindest ein Teil der Vorrichtung eine Beschichtung umfasst.

9. Vorrichtung nach einem der vorhergehenden Ansprüche, worin zumindest ein Teil der Vorrichtung ferner ein röntgenopakes Material umfasst.

10. Vorrichtung nach einem der vorhergehenden Ansprüche, worin die Vorrichtung eine Beschichtung umfasst, die zumindest das erste verriegelnde Verbindungsglied oder das zweite verriegelnde Verbindungsglied bedeckt, um eine glatte Außenfläche zu formen.

11. Vorrichtung nach einem der vorhergehenden Ansprüche, worin die Vorrichtung einen Führungsdraht umfasst.

12. Vorrichtung nach einem der vorhergehenden Ansprüche, worin die Vorrichtung so konfiguriert ist, dass sie im Magen-Darm-Trakt eines Patienten angeordnet werden kann.

13. Längliches medizinisches System, das Folgendes umfasst: einen Katheter mit einem proximalen Abschnitt und einem distalen Abschnitt; und einen Führungsdraht nach Anspruch 11, der sich vom distalen Abschnitt des Katheters aus erstreckt.

## Revendications

1. Dispositif médical allongé (20) configuré pour naviguer dans un lumen, le dispositif comprenant :
une tige allongée (22) comprenant une partie proximale (26) et une partie distale (28), la partie distale comprenant une première pièce de liaison (29) et
une partie en boucle (30) comprenant une deuxième pièce de liaison (33) configurée pour être reliée à la première pièce de liaison de la tige,
la deuxième pièce de liaison étant configurée pour être reliée de façon libérable à la première pièce de liaison dans une configuration de liaison encliquetée.

2. Dispositif selon la revendication 1, dans lequel la première pièce de liaison ou la deuxième pièce de liaison comprennent une saillie et l'autre pièce de liaison comprend un réceptacle.

3. Dispositif selon la revendication 2, dans lequel la saillie et le réceptacle ont une forme sphérique.

4. Dispositif selon l'une quelconque des revendications précédentes, dans lequel la partie en boucle comprend une zone de pliage.

5. Dispositif selon l'une quelconque des revendications précédentes, dans lequel au moins une partie du dispositif comprend un matériau bioabsorbable.

6. Dispositif selon l'une quelconque des revendications précédentes, dans lequel au moins une partie du dispositif est constituée d'un polymère.

7. Dispositif selon l'une quelconque des revendications précédentes, dans lequel au moins une partie du dispositif est constituée de nitinol.

8. Dispositif selon l'une quelconque des revendications précédentes, dans lequel au moins une partie du dispositif comprend de plus un revêtement.

9. Dispositif selon l'une quelconque des revendications précédentes, dans lequel au moins une partie du dispositif est constituée d'un matériau opaque aux rayonnements.

10. Dispositif selon l'une quelconque des revendications précédentes, le dispositif comprenant un revêtement qui couvre au moins la première pièce de liaison ou la deuxième pièce de liaison afin de former une surface externe lisse.

11. Dispositif selon l'une quelconque des revendications précédentes, le dispositif comprenant un passe-fil.

12. Dispositif selon l'une quelconque des revendications précédentes, le dispositif étant configuré pour être placé dans le tractus gastrointestinal d'un patient.

13. Dispositif médical allongé comprenant :
un cathéter qui comprend une partie proximale et une partie distale et
un passe-fil selon la revendication 11, qui déborde de la partie distale du cathéter.
